# EUROPEAN PATENT SPECIFICATION

(11) **EP 1 124 550 B1**
(45) Date of publication and mention of the grant of the patent: **16.07.2008**
(21) Application number: 99945197.4
(22) Date of filing: 30.08.1999
(51) Int. Cl.: A61K 31/34, A61K 31/375, A61K 31/715, A61K 31/365, A61P 35/00

(54) **USES AND COMPOSITIONS FOR SELECTIVE CANCER CHEMOTHERAPY**
VERWENDUNGEN UND ZUSAMMENSETZUNGEN FÜR SELEKTIVE KREBS-CHEMOTHERAPIE
UTILISATIONS ET COMPOSITIONS POUR CHIMIOTHERAPIE ANTICANCEREUSE SELECTIVE

(43) Date of publication of application: 22.08.2001
(73) Proprietor: The Ester C Company, Prescott AZ 86301 (US)
(72) Inventor: JARIWALLA, Raxit, J., Saratoga, CA 95070 (US)
(74) Representative: Brereton, Paul Arthur
(86) International application number: PCT/US1999/019449
(87) International publication number: WO 2001/015692

(56) References cited:
- EP-A- 0 369 079
- WO-A-90/12571
- WO-A-99/39580
- US-A- 4 822 816
- US-A- 5 626 883
- US-A- 5 869 116
- LEUNG, PING Y. ET AL: "Cytotoxic effect of ascorbate and its derivatives on cultured malignant and nonmalignant cell lines" ANTICANCER RES. (1993), 13(2), 475-80 , XP001070180
- LIOTTI, F. S. ET AL.: "Effects of ascorbic-acid and dehydroascorbic-acid on the multiplication of tumor ascites cells in-vitro" JOURNAL OF CANCER RESEARCH AND CLINICAL ONCOLOGY, vol. 108, no. 2, 1984, page 230-232 XP001069999
- GERSHOFF, S.N.: "Vitamin C (ascorbic acid): new roles, new requirements?" NUTRITION REVIEWS, vol. 51, no. 11, - November 1993 (1993-11) pages 313-326, XP001058037
- "Ester-C complex" NATURAL HEALTH CONSULTANTS, [Online] XP002193240 Retrieved from the Internet: <URL:http://www.naturalhealthconsult.com/M onographs/esterC.html> [retrieved on 2002-03-05]

## Description

### Field of the Invention

This invention relates to tumor-cytotoxic chemotherapeutic methods.

In another aspect the invention relates to tumor-cytotoxic chemotherapeutic compositions.

More particularly the invention concerns tumor-cytotoxic chemotherapeutic methods and compositions for treating cancers in a human host.

### Background of the Invention

Tumor cytotoxic chemotherapeutic agents preferentially induce death (apoptosis) of malignant cells. Because of similarities between normal and malignant cells, both being born of the same host, a chemotherapeutic dose which induces apoptosis of tumor cells can also be toxic to normal cells. In order to effect a remission, the tumor-cytotoxic agent must often push the limits of acceptable side effects. Ideally, the tumor-cytotoxic agent should be "selective", i.e., there should be a large gap between the lower dose required to induce tumor cell death, for efficacy as a tumor-cytotoxic chemotherapeutic agent, and the higher dose which is toxic to the patient's normal cells.

The adverse side-effects of chemotherapy may include hair loss, nausea and vomiting, cardiac toxicity and secondary cancers. One of the most common side-effect toxic manifestations of many cytotoxic agents is bone marrow suppression, which can lead to immune suppression and hematopoietic dysfunctions. Because infectious complications are one of the major causes of death in cancer patients, it would be highly desirable to provide non-toxic tumor-cytotoxic chemotherapeutic compositions and methods without immunosuppressive side effects.

Compounds having vitamin C activity, e.g., ascorbic acid and ascorbate derivatives, are not immunosuppressive, but are effective intravenous cytotoxic chemotherapeutic agents against a wide variety of cancers. Riordan et al., Medical Hypotheses, 1995, 44, 207-213. However, there is no vitamin C storage mechanism in human tissues and it is all metabolized and/or excreted. Further, because of gastrointestinal complications, it is difficult to establish and maintain high serum levels of vitamin C by oral administration of ascorbic acid. Thus, it is generally considered necessary to administer ascorbic acid intravenously in order to establish and maintain plasma levels sufficiently high to achieve cytotoxicity. Therefore, it would be extremely advantageous to provide tumor chemotherapeutic compositions, containing forms of vitamin C other than ascorbic acid, which can be orally administered in doses sufficiently high to establish and maintain a tumor cytotoxic level of serum vitamin C.

However, because even vitamin C can be toxic to normal human cells if the plasma concentration is sufficiently high, it would also be highly desirable to provide selective vitamin C tumor chemotherapeutic compositions in oral or intravenous dosage forms, which achieve tumor cell apoptosis at lower plasma concentrations than those required for ascorbic acid to induce tumor cell apoptosis. Because the tumor cytotoxic concentration of vitamin C administered from such dosage forms would be lower, it would be more feasible to establish and maintain a chemotherapeutically effective plasma concentration at a level which would be below the vitamin C plasma apoptosis level for normal cells.

### The Prior Art

As reviewed by Cameron et al. (Cancer Res., 39:663-81 (1979)) some clinical trials have shown significant increases in survival times of cancer patients receiving vitamin C.

Elvin et al. (Eur. J. Cancer Clin. Oncol. 17(7):759-65 (1981)) reported that adducts of ascorbic acid with aldehydes such as methylglyoxal and acetylacrolein inhibit growth of Ehrlich ascites carcinoma in mice.

EP-A-0086544 proposes uses of ketals and acetals of ascorbic acid as angiogenesis-inhibiting agents. (Angiogenesis refers to the process of new blood vessel development, the proliferation of new blood vessels being involved in tumor growth.)

EP-A-0148094 and U.S. Patent 5,032,610 propose that orally administered or intravenously administered 5,6-O-benzylidene-L-ascorbic acid and salts thereof and mixtures thereof with L-ascorbic acid and salts thereof exhibit anticancer properties.

Concomitant administration of 3-amino-1,2,4-triazole enhances the cytotoxicity of ascorbic acid to Ehrlich ascites tumor cells and the addition of vitamin K3 (menadione sodium bisulfite) appears to increase preferential tumor cytotoxicity of ascorbic acid. Benande et al., Oncology, 23:33-43 (1969).

Also, prior workers have shown that catalytic concentrations of Cu²⁺ increased the preferential toxicity of ascorbic acid for several malignant melanoma cell lines, including four human-derived lines. Bram et al., Nature 284: 629-631(1980).

Several leukemic, pre-leukemic and myeloma progenitor cells derived from human patients were reported to be sensitive to ascorbic acid concentrations attainable *in vivo*, without any toxicity to normal hemopoietic cells. Park et al., Cancer Res. 4:1062-65 (1980); Am.J.Clin.Nutr. 54:1241S-46S (1991).

WO 99 39580 describes Vitamin C compositions which comprise at least one mineral ascorbate dissolved in at least one pharmacologically acceptable liquid organic polyol solvent.

### Description of the Drawings

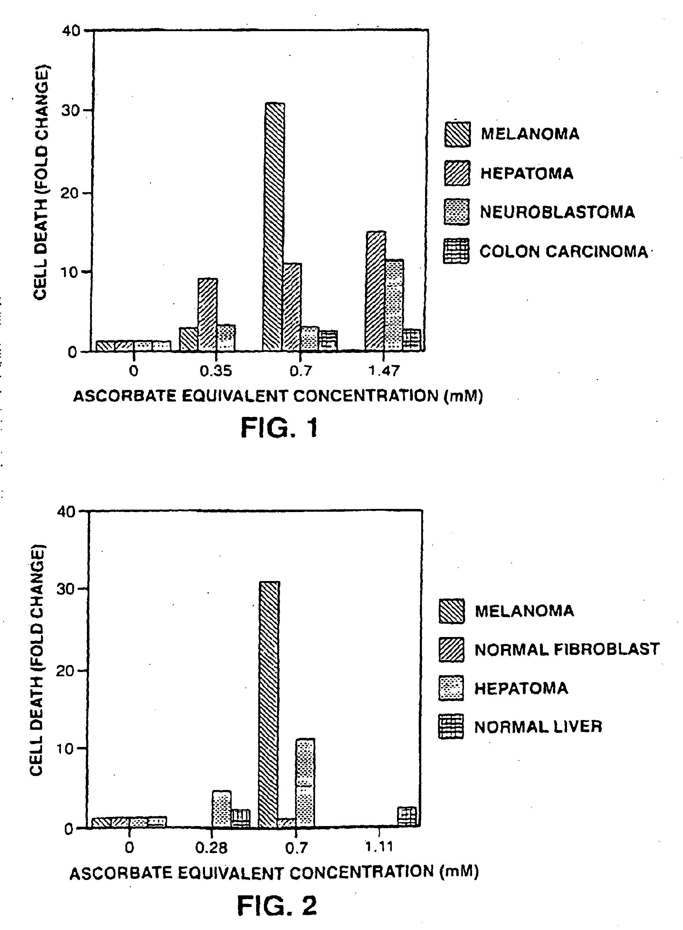

Fig. 1 is a bar graph which illustrates the apoptosis of various tumor cell-types by the "mineral ascorbate plus metabolite" composition employed in the practice of the preferred invention, as illustrated by Test 1.
Fig. 2 is a similar bar graph which illustrates the selectivity of apoptosis of various tumor cell-types over normal cells by the "mineral ascorbate plus metabolites" composition employed in the preferred practice of the invention, as illustrated by Test 1.

### Brief Description of the Invention

The present invention is the use of a composition as defined in claim 1 in preparing a cancer chemotherapy composition.

### The Preferred Embodiments of the invention

The components of the above-described chemotherapeutic composition are simply mixed together in appropriate proportions. The exact proportions are not highly critical. Operable and optimum proportions can be determined and varied within limits which can be determined without undue experimentation by those skilled in the art, e.g., by employing *in vitro* tests such as those described below. Alternatively, in accordance with the presently preferred embodiment of the invention, suitable mineral ascorbate-metabolite compositions, containing these components in appropriate proportions, are commercially available under the registered trademark ESTER-C® from Inter-Cal Corporation, Prescott, Arizona, USA. These compositions are further described in United States Patents 4,822,816; 4,968,716; and 5,070,085.

The cytotoxically effective vitamin C plasma concentration provided by the chemotherapeutic methods and compositions of the invention will vary according to the specific type of tumor cells being treated and can be determined by *in vitro* tests such as those described below, animal tests and human *in vivo* trials, in accordance with art-recognized techniques.

The chemotherapeutic compositions of the invention are formulated for intravenous administration by inclusion of the mineral ascorbate and vitamin C metabolite components in a pharmaceutically acceptable intravenous carrier, i.e., a sterile, non-toxic solution of the components in a carrier, formulated to provide appropriate osmolality, pH, etc., in accordance with art-recognized techniques. For example, Ringer's Lactate is an appropriate intravenous carrier.

The concentration of vitamin C in the intravenous carrier can be varied within wide limits to suit the requirements of treatment. For example, when it is desired to establish an ascorbic acid equivalent plasma concentration in the range 150-200 mg/dL, an appropriate dosage for an 8-hour, 1000 cc infusion is 100-150 mg of ascorbate provided by the mineral salt. It may be required to repeat such infusions several times before reaching and maintaining the desired plasma concentration, depending on the capacity of the patient's system for ascorbate destruction, elimination or excretion.

It is also possible to employ oral dosage forms containing the mineral ascorbate/vitamin C metabolite compositions to establish initial plasma concentrations of these compositions which are effective to induce apoptosis in some forms of tumors. According to my present information, at oral dosages in the range of approximately 12-15 grams of ascorbate per day, a plasma level (AA equivalent) of approximately 5 mg/dl is attainable, which is sufficient to induce selective apoptosis of melanoma and hepatoma cells.

Moreover, once a selective tumor apoptosis-inducing plasma concentration is obtained by intravenous administration, that concentration can be maintained by administration of oral dosage forms or by a combination of oral and intravenous administration.

### EXAMPLES

Several tumor cell lines and corresponding normal non-malignant cell lines are tested for apoptosis by Ester-C® (Calcium Ascorbate plus metabolite) versus four other test compositions, calcium ascorbate (CA) alone, calcium threonate (CT) alone and calcium ascorbate plus calcium threonate (CA+CT) and sterile water (sH₂O).

### EXAMPLE 1

### Test Procedures

The cell lines are:

| | |
|---|---|
| Malme-3M | Melanoma, Human (ATCC No. HTB-64) |
| Malme-3 | Normal Human Skin Fibroblasts (ATCC No. HTB-102) |
| SK-Hep-1 | Liver adenocarcinoma, Human (ATCC No. HTB-52) |
| WRL | Normal Human Liver Cells (ATCC No. CL-98) |
| SK-N-MC | Neuroblastoma, Human (ATCC No. HTB-10) |
| T-84 | Colon Carcinoma, Human (ATCC No. CCL-248) |

Stock cells are grown in the growth media, as follows:

| Cell Line | Growth Media |
|---|---|
| SK-Hep-1, SK-N-MC and WRY 88 | Eagle's Minimal Essential Medium in Earle's salts supplemented with 2mM L-glutamine, 1mM sodium pyruvate, 10% fetal bovine serum (FBS) and antibiotics (penicillin, streptomycin, amphotericin B) |
| | |
| Malme-3 and Malme 3-M | McCoy's medium with L-glutamine, 15% FBS and antibiotics |
| | |
| T-84 | 1:1 mixture of Dulbecco's modified MEM and Ham's F-12 medium with L-Glutamine, pyridoxal hydrochloride, 25 mM Hepes plus 5% FBS and antibiotics |

All cultures are maintained at 37 C in a humidified atmosphere of 5%CO₂/95% air. Media and culture reagents are obtained from Life Technologies (Gibco/BRL, Long Island, NY). FBS is obtained from Hyclone Labs (Logan, UT).

Test materials are obtained from Inter-Cal Corporation (Prescott, AZ), as follows:

| | |
|---|---|
| Test 1 | Ester-C® Mineral Ascorbate (see below) |
| | |
| Test 2 | Calcium Ascorbate (USP Grade), 82.15% ascorbic acid (AA) equivalent |
| | |
| Test 3 | Calcium Threonate, 87.08% L-threonic acid (TA) equivalent |
| | |
| Test 4 | Calcium Ascorbate (U.S.P.) + Calcium U.S.P., 81.21% AA, 1% TA equivalent |
| | |
| Test 5 | Ascorbic Acid |

The Test 1 material contains the following by laboratory analysis:

| | |
|---|---|
| Calcium Ascorbate | 78.4% AA equivalent |
| Calcium Threonate | .9% TA equivalent |
| Other AA Metabolites¹ | 10.4% AA equivalent |
| Water of Crystallization | Balance |

| | |
|---|---|
| ¹ aldonic acids, the aldono-lactones, aldono-lactides and non-toxic metal salts of aldonic acids, dehydroascorbic acid, threose, erythreose, 4-hydroxy-5-methyl-3(2H)-furanone, 3-hydroxykojic acid and 5-hydroxymaltol. | |

Ascorbic Acid (tissue culture grade) is obtained from Sigma Chemical Co. (St. Louis, MO). Control compositions consist of growth medium, Ringer's Solution or sterile water, as appropriate.

All working solutions are prepared from master stocks immediately before use. A 60 mM master stock solution of AA is prepared in serum-free growth medium and stored at -15 C. Working solutions are made from 10X strength stock solutions by dilution in growth medium. A 30mM (1gm/%) stock of calcium threonate is made in Ringer's solution (Fay and Verlangieri, Life Sciences, **49**:1377 (1991)) or warm sterile water. Working solutions are made as 1X strength stock (in Ringer's solution) or as 10X stock (in sH₂O), depending on the nature of the treatment, i.e., short-term versus continuous, (see below).

For evaluation of the Test 1 material, 1-1.3% master stock solution of the Test material is prepared in warm sterile water. Working stock solutions (10X strength) are made in sterile water immediately before use. For comparative evaluation, stock solutions of Test 2 and Test 4 solutions are prepared in sterile water, normalized to contain AA equivalents identical to the Test 1 stock. These stocks are stored at room temperature for use in evaluations.

### Example 2

### Treatment of Cell Cultures with Ascorbic Acid and/or Calcium Threonate

0.25-1.0 x10⁵ cells of tumor-derived or normal liver cell lines are seeded and cultured in individual wells of a 24-well cluster plate in the presence of increasing concentrations of freshly prepared supplement consisting of ascorbic acid (AA) or calcium ascorbate (CA). Cultures are re-fed periodically with additions of respective supplements, with or without medium change as indicated. Controls consist of cells receiving growth medium without added supplement.

For treatment with calcium threonate (CT), cells are allowed to attach by overnight incubation. The following day, threonate treatment is initiated using one of two protocols.

In one protocol (short exposure), monolayers are washed and exposed directly to 1 mL/well of 7.5-30 mM threonate (prepared in Ringer's solution) for brief periods at 37 C as described by Fay and Verlangieri (referenced above). Controls are exposed to 1 mL/well of Ringer's solution alone for similar intervals. After exposure, the solution is removed and replaced by growth medium.

In the other protocol (continuous exposure), one-tenth volume of 10X strength threonate (prepared in sterile water) is added to the culture medium and the incubation continued at 37 C. This same treatment is repeated by daily additions of fresh working solution.

### Example 3

### Treatment of Cell Cultures With Ascorbic Acid Plus Calcium Threonate

Cells are treated with calcium threonate (CT), as in Example 2, for sixty minutes at 37C followed by addition of ascorbic acid (AA) in Ringer's solution and continuous incubation for thirty minutes. At the end of the treatment period, the solution is removed and replaced by 1 mL/well of growth medium.

### Example 4

### Treatment of Cell Cultures with Test 1 (Ester-C® Calcium Ascorbate Plus Metabolites) and Test 4 (Calcium Ascorbate plus Calcium Threonate)

Subconfluent monolayers of cell cultures, seeded in 24-well cluster plates, are supplemented with one-tenth volume of working stock solutions to obtain final Test 1 concentration in the range 0.006 to 0.06% (corresponding to 0.28 mM-2.8mM ascorbic acid equivalents). Parallel sets of wells are treated similarly with stock solutions of CA + CT and CA alone, containing the same equivalents of AA as Test 1. Control cultures are treated with an equivalent volume of sterile water. Periodic treatment with these compositions is repeated at 1-2 day intervals by direct addition of fresh solutions (without change of growth medium).

### Example 5

### Assay of Cell Survival and Cell Death (Apoptosis)

Cell survival following treatment is assessed at predetermined intervals by taking viable cell counts using a Neubauer haemocytometer. Viable cells are scored as those capable excluding trypan blue as previously described (Harakeh and Jariwalla, Am.J.Clin.Nutr. **54**:1231S-1235S (1991)). The data are used to plot viable cell culture (# of cells/ml) against the concentration of Test solutions to evaluate the effect on cell survival.

The induction of apoptosis following treatment of various tumor cell types with the Test compositions and controls described in Example 1 is evaluated using an enzyme-linked immunoassay ("ELISA") developed by Boehringer-Mannheim (Indianapolis, IN). This assay specifically screens and detects histone-associated DNA complexes (nucleosomal fragments) appearing in the cytoplasm of treated cells relative to that in untreated controls. The presence and level of nucleosomal fragments in cytoplasmic lysates after different treatments is determined using the procedure specified in the cell-death detection ELISA kit, supplied by Boehringer-Mannheim.

Briefly, the ELISA assay is carried out as follows. At different intervals following treatment with the Test compositions, medium is aspirated and cell membranes are lysed by incubation with 200-500 µL of lysis solution for 30 minutes at room temperature. Cell lysate is collected in an Eppendorf tube and centrifuged at 2500 rpm for 10 minutes to separate the nuclear fraction. An aliquot of the supernatant containing the cytoplasmic fraction is used to quantify the nucleosomal fragments by photometric detection at 410 nm in a micro plate reader. Data are processed as follows: The mean absorbance at 410 nm is plotted against the concentration of the Test compound to compare the relative apoptosis-inducing doses determined for each treatment and compared. The minimal apoptosis-inducing dose is defined as that required to cause 2-fold change in apoptosis (i.e., nucleoprotein level) over that in the untreated control. Maximal apoptosis is defined as the maximal fold change in apoptosis over control.

**TABLE 1**

| **SUMMARY OF DATA** | | | | | | |
|---|---|---|---|---|---|---|
| | | **MINIMUM APOPTOTIC DOSE** | | **MAXIMUM FOLD INCREASE IN APOPTOSIS** | | |
| **COMPOSITION** | **CELL LINE** | **DOSE CONC. (%)** | **# OF TREATMENTS** | **DOSE CONC. (%)** | **# OF TREATMENTS** | **MAXIMUM FOLD INCREASE IN APOPTOSIS** |
| [Calcium | Malme-3 | 0.025 | 2 | 0.033 | 2 | 3.58 |
| Ascorbate + | Malme-3M | 0.006 | 1 | 0.025 | 2 | 116 |
| Metabolites] | WRL-68 | 0.006-0.012 | 3 | 0.025 | 2 | 2.04 |
| | SK-Hep-1 | 0.006 | 3 | 0.033 | 3 | 14.9 |
| | SK-N-MC | 0.008 | 2 | 0.033 | 4 | 13.6 |
| | T-84 | 0.015 | 3 | 0.02-0.03 | 4 | 4 |
| [Calcium | Malme-3 | 0.025 | 2 | 0.025 | 2 | 2.16 |
| Ascorbate + | Malme-3M | 0.012 | 2 | 0.025 | 2 | 65.7 |
| Calcium | WRL-68 | >0.05 | 3 | 0.0125 | 2 | 1.68 |
| Threonate] | SK-Hep- | 0.006 | 3 | 0.033 | 3 | 9.31 |
| | SK-N-MC | 0.015 | 4 | 0.033 | 4 | 7.02 |
| | T-84 | >0.06 | 3 | 0.033 | 3 | 1.5 |
| Calcium | Malme-3 | 0.025 | 2 | 0.025 | 2 | 2.16 |
| Ascorbate | Malme-3M | 0.012 | 2 | 0.025 | 2 | 92.8 |
| | WRL-68 | 0.012 | 3 | 0.012 | 3 | 2.22 |
| | SK-Hep-1 | 0.006 | 3 | 0.033 | 3 | 12.04 |
| | SK-N-MC | 0.015 | 4 | 0.015 | 4 | 5.03 |
| | T-84 | >0.06 | 3 | 0.033 | 3 | 1.77 |

In Table 1, above, the "Minimum Apoptotic Dose" is the concentration of the Composition required to cause a 2-fold change in apoptosis over the control. The "Number of Treatments" is the total number of treatments with the Composition. The "Maximum Fold Increase in Apoptosis" is the maximal fold change in apoptosis over the control and the "Maximum Dose" is the concentration of the Composition which induces the "Maximum Fold Apoptosis" compared to the control.

### CONCLUSIONS

The results of tests described above lead to the following conclusions:

The mineral ascorbate/vitamin C metabolite compositions illustrated by the Test 1 composition induce selective cell death (apoptosis) of diverse tumor cell-types in a dose-dependent fashion.

Mineral ascorbate/vitamin C metobolite compositions (as illustrated by the Test 1 composition) achieve apoptosis, i.e., minimum two-fold increase in cell death rate, at lower concentrations (AA equivalent) than is required to achieve such decrease with either mineral ascorbate alone (as illustrated by the Test 2 composition) or with ascorbic acid alone.

The maximal level of apoptosis achievable with mineral ascorbate/vitamin C metabolite compositions (as illustrated by the Test 1 composition) against specific cell types, is higher than achievable with mineral ascorbate or ascorbic acid alone.

The AA equivalent concentration of mineral ascorbate/vitamin C metabolite compositions (as illustrated by the Test 1 composition) required to induce apoptosis in tumor cells is lower than for normal cells, and the magnitude of cell death in normal cells is considerably smaller than in tumor cells.

Treatment of cell cultures with ascorbic acid (AA) and/or calcium threonate (CT) (as illustrated in Example 2) produced selective dose-dependent cell death (apoptosis) in hepatoma and melanoma cells as compared to their respective normal cellular counterparts.

Pretreatment of hepatoma cells with CT followed by application of AA induced higher level of cellular apoptosis than corresponding dose of AA or CT alone.

## Claims

1. The use in the preparation of a cancer chemotherapy composition of a composition comprising:
(a) a plasma-soluble mineral ascorbate; and
(b) one or more vitamin C metabolites selected from the group consisting of:
(i) aldonic acids, and the aldono-lactones, aldono-lactides and non-toxic metal salts thereof, and
(ii) dehydroascorbic acid, threose, erythreose, 4-hydroxy-5-methyl-3(2H)-furanone, 3-hydroxykojic acid and 5-hydroxymaltol.

2. The use according to claim 1 in which the composition includes approximately 78.4% AA equivalent of calcium ascorbate, 0.9% TA equivalent of calcium theonate, 10.4% AA equivalent of other AA metabolites and the balance in water of crystalization.

3. The use according to claim 1 or 2 including a pharmacologically acceptable intravenous carrier.

4. The use according to claim 1, 2 or 3 in which the composition is for use at a dose of 12 to 15 g of ascorbate per day.

5. A compound for use in cancer chemotherapy comprising:
(a) a plasma soluble mineral ascorbate; and
(b) one or more vitamin C metabolites selected from the group consisting of:
(i) aldonic acids, and the aldono-lactones, aldono-lactides and non-toxic metal salts thereof; and
(ii) dehydroascorbic acid, threose, erythreose, 4-hydroxy-5-methyl-3(2H)-furanone, 3-hydroxykojic acid and 5-hydroxymaltol.

6. A compound according to claim 5 which includes approximately 78.4% AA equivalent of calcium ascorbate, 0.9% TA equivalent of calcium threonate, 10.4% AA equivalent of other AA metabolites and the balance in water of crystallization.

7. A compound according to claim 5 or 6 including a pharmacologically acceptable intravenous carrier.

8. A compound according to any of claims 5 to 7 for use at a dose of 12 to 15 g of ascorbate per day.

## Patentansprüche

1. Verwendung einer Zusammensetzung zur Herstellung einer Krebs-Chemotherapiezusammensetzung, die Folgendes umfasst:
(a) ein plasmalösliches Mineralascorbat und
(b) einen oder mehrere Vitamin-C-Metabolite, ausgewählt aus der Gruppe bestehend aus:
(i) Aldonsäuren und den Aldono-Lactonen, Aldono-Lactiden und nicht toxischen Metallsalzen davon und
(ii) Dehydroascorbinsäure, Threose, Erythreose, 4-Hydroxy-5-metyhl-3(2H)-furanon, 3-Hydroxykojisäure und 5-Hydroxymaltol.

2. Verwendung nach Anspruch 1, wobei die Zusammensetzung etwa 78,4 % AA-Äquivalent von Calciumascorbat, 0,9 % TA-Äquivalent von Calciumtheonat, 10,4 % AA-Äquivalent anderer AA-Metabolite umfasst und der Rest Kristallwasser ist.

3. Verwendung nach Anspruch 1 oder 2, umfassend einen pharmakologisch akzeptablen intravenösen Träger.

4. Verwendung nach Anspruch 1, 2 oder 3, wobei die Zusammensetzung in einer Dosis von 12 bis 15 g Ascorbat pro Tag verwendet wird.

## Revendications

1. Utilisation dans la préparation d'une composition de chimiothérapie pour le cancer, d'une composition comprenant:
(a) un ascorbate minéral soluble dans le plasma; et
(b) un ou plusieurs métabolites de vitamine C sélectionnés parmi le groupe consistant en :
(i) acides aldoniques et les aldonolactones, aldonolactides et des sels métalliques non toxiques de ceux-ci, et
(ii) acide déshydroascorbique, thréose, érythréose, 4-hydroxy-5-méthyl-3(2H)-furanone, acide 3-hydroxykojique et 5-hydroxymaltol.

2. L'utilisation selon la revendication 1, dans laquelle la composition comprend approximativement 78,4% en équivalent AA d'ascorbate de calcium, 0,9% en équivalent TA de thréonate de calcium, 10,4% en équivalent AA d'autres métabolites d'AA et le reste en eau de cristallisation.

3. L'utilisation selon la revendication 1 ou 2, comprenant un excipient intraveineux pharmacologiquement acceptable.

4. L'utilisation selon la revendication 1, 2 ou 3, dans laquelle la composition doit être utilisée à une dose de 12 à 15 g d'ascorbate par jour.
